(19) Europäisches Patentamt
European Patent Office
Office européen des brevets

(11) Publication number: 0 232 250 B1

(12) # EUROPEAN PATENT SPECIFICATION

(45) Date of publication of patent specification: 31.01.90

(51) Int. Cl.$^5$: A 61 L 2/18

(21) Application number: 85903776.4

(22) Date of filing: 22.07.85

(86) International application number: PCT/US85/01393

(87) International publication number: WO 87/00437 29.01.87 Gazette 87/03

(54) METHODS AND SOLUTION FOR DESINFECTING AND PRESERVING CONTACT LENSES.

(43) Date of publication of application: 19.08.87 Bulletin 87/34

(45) Publication of the grant of the patent: 31.01.90 Bulletin 90/05

(84) Designated Contracting States: AT BE CH DE FR GB IT LI LU NL SE

(56) References cited:
EP-A-0 055 515
US-A-2 684 924
US-A-3 689 673
US-A-4 354 952

The file contains technical information submitted after the application was filed and not included in this specification

(73) Proprietor: BAUSCH & LOMB INCORPORATED
1400 North Goodman Street
Rochester New York 14609 (US)

(72) Inventor: OGNUBIYI, Lai
9 Morningview Drive
Fairport, NY 14450 (US)
Inventor: SCOTT, Francis, L.
6706-B East Cefdar Avenue
Denver Colorado 80224 (US)
Inventor: SMITH, Francis, X.
4281 Downs Road
Walworth, NY 14563 (US)

(74) Representative: Allam, Peter Clerk et al
LLOYD WISE, TREGEAR & CO. Norman House
105-109 Strand
London WC2R 0AE (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 232 250 B1

## Description

Background of the Invention

This invention relates generally to improved methods for disinfecting and/or preserving contact lenses, and to improved contact lens car solutions. The solutions contain monomeric biguanides as their principal active disinfecting agent, perform with a low level of residual binding or concentrating onto lens surfaces and exhibit low toxicity levels. The solutions described herein are compatible for use with other germicidal agents for solution preservation, as well as for complementing or broadening the spectrum of microbicidal activity. In some instances, lower concentrations of such agents may be used, further reducing the risk of residual build-up on soft contact lenses and incidents of eye tissue irritation and inflammation.

Generally, contact lenses in wide use fall into two categories: the hard or rigid corneal type lenses formed from materials prepared by polymerization of acrylic esters, such as polymethyl methacrylate (PMMA), and gel, hydrogel or soft type lenses made of polymerized hydrophilic or hydrophobic monomers, such as 2-hydroxyethyl methacrylate (HEMA). The hard acrylic type contact lenses are characterized by low water vapor diffusion constants, resistance to the effects of light, oxygen and hydrolysis and absorb only minor amounts of aqueous fluids. Because of the durability of hard contact lenses coupled with their tendency not to absorb appreciable amounts of water, the selection of suitable disinfecting agents, cleaning agents or other lens care compounds is relatively non-critical.

However, unlike hard lenses, soft type contact lenses and certain of the newer gas permeable hard contact lenses have a tendency to bind and concentrate significantly more fluids, environmental pollutants, water impurities, as well as antimicrobial agents and other active ingredients commonly found in lens care solutions. In most instances, the low levels of such ingredients in lens care solutions does not lead to eye tissue irritation when used properly. Nevertheless, because of the inherent binding action of protein deposits and soft lens materials disinfecting agents and preservatives tend to build up on lens surfaces, and become concentrated to potentially hazardous levels, such that when released can cause corneal inflammation and other eye tissue irritation.

Previous efforts to alleviate the problem of binding and concentrating disinfectants and preservatives onto contact lens surfaces, and reducing the potential for eye tissue irritation have not been totally satisfactory. For example, in spite of low toxicity levels not all disinfectants are compatible for use with all types of contact lenses. Many hard lens disinfecting and preservative solutions contain benzalkonium chloride or chlorobutanol. Although they are effective antibacterial agents, there use can result in a loss of lens hydrophilic properties, cause solution instability or may even lack compatibility with certain types of hard lenses, e.g. high silicon content.

Other antibacterial agents were found to be more compatible with contact lenses and exhibit less binding on lens surfaces. US—A—4,361,548 discloses a contact lens disinfectant and preservative containing dilute aqueous solutions of dimethyldiallylammonium chloride (DMDAAC) wherein amounts of DMDAAC as low as 0.00001 percent by weight are employed when an enhancer, such as thiomersal, sorbic acid or phenylmercuric salt are used therewith. Although lens binding and concomitant eye tissue irritation with DMDAAC were reduced, it was found in some users to be above desirable clinical levels.

Other efforts to reduce or eliminate soft lens binding have led to the use of anti-binding or detoxifying agents, like polyvinyl pyrrolidone (PVP) and polyvinyl alcohol (PVA). However, these polymers alone were found to be ineffective, for the most part, in reducing lens binding and eye tissue irritation.

Heretofore, monomeric biguanides have been used in lens disinfecting and preservative solutions. For example, the biguanide chlorhexidine was reported by B. S. Plant et al in the *J. Pharm. Pharmacol.*, 32, 453—459 (1980) suggesting its use as a disinfectant with soft contact lenses. It was discovered, however, that chlorhexidine was absorbed to some extent by such lenses and was therefore not an ideal agent for such systems. One solution to this problem is suggested by US—A—4,354,952 which discloses very dilute disinfecting and cleaning solutions containing chlorhexidine or its salts in combination with certain amphoteric and non-ionic surfactants. Although these solutions are effective in reducing binding properties, their antimicrobial activity may be diminished when used with certain amphoteric surfactants. In the case of chlorhexidine, it was found that if not used in the proper ratio the surfactant and disinfectant will precipitate unless a combination of surfactants are employed.

While it has been reported that more conventional chlorhexidine-containing disinfectant solutions bind to soft contact lenses seven times less than benzalkonium chloride, the presence of proteinaceous oily, tear-film deposits can double the amount of chlorhexidine absorbed over that of clean lenses.

It has now been found that certain monomeric biguanides demonstrate unusually low levels of lens absorption, binding and toxic response. This is rather unexpected in view of the fact that past efforts to solve the aforestated problem have generally shown that polymers, and usually higher molecular weight materials, provided the lowest levels of lens absorption and binding activity. That is to say, heretofore, it was generally held that polymeric type disinfectants offered the most probable rationale for solving the problem of lens binding and eye tissue irritation, since their molecules were less likely to penetrate lens micropores than their monomeric counterparts. Hence, the present invention provides for an improved and unexpected new means for treating contact lenses. The solutions are compatible for use with both hard and soft type lenses, and are adaptable for use with virtually any of the commonly known disinfecting techniques, including "cold" soaking under ambient temperature conditions, as well as with high

temperature disinfecting methods. The disinfecting and preservative solutions employed according to the invention described herein are especially noteworthy for their wide spectrum of bactericidal and fungicidal activity, at low concentrations coupled with low toxicity levels and reduced affinity for binding and concentrating when used with soft type contact lenses.

More particularly, in accordance with this invention, there is provided a method for disinfecting and/or preserving contact lenses which comprises contacting said lenses with an antimicrobial amount of a solution of a biguanide or water-soluble salt thereof having the following general formula I:

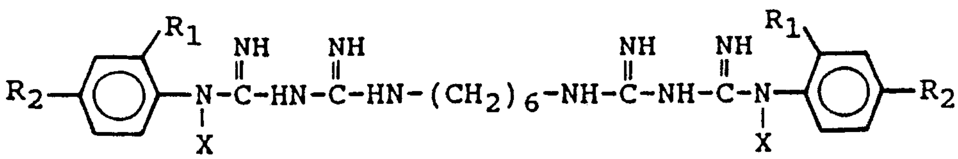

(I)

wherein $R_1$ and $R_2$ are independently hydrogen or halogen, and X is hydrogen or $C_1$—$C_3$ alkyl, provided that when $R_2$ is halogen then $R_1$ is hydrogen and X is $C_1$—$C_3$ alkyl.

The present invention also provides a contact lens cleaning or disinfecting solution comprising a biguanide of formula I, as defined above, or a water-soluble salt thereof.

The above biguanides of formula I are utilized in lens disinfecting and/or preservative solutions in microbially effective concentrations. The antibacterial-antifungal action of the biguanide-containing solutions described herein may also be supplemented by the addition of other germicidal agents. Because the overall germicidal activity of such combinations may in some instances be greater than when each is used separately, the concentration of total disinfectant in solution under such circumstances can be lowered, further reducing the potential for binding, concentrating and adverse toxic effects.

The solutions of this invention may be used to disinfect and/or preserve most contact lenses, including hard and soft lenses, as well as the newer hard gas permeable type contact lenses, such as described in US—A—4,327,203. The term "soft contact lens" as used herein generally refers to contact lenses which readily flex under small amounts of force and return to their original shape when the force is released. Typically, soft contact lenses are formulated from poly(hydroxyethyl methacrylate) which has been, in the preferred formulations, cross-linked with ethylene glycol dimethacrylate. For convenience, this polymer is generally known as PHEMA. Soft contact lenses are also made for silicon polymers cross-linked, for example, with dimethyl polysiloxane. Conventional "hard contact lenses", which cover only the cornea of the eye, usually consist of poly(methyl methacrylate) cross-linked with ethylene glycol dimethacrylate.

The disinfecting solutions employed in the present invention are effective at low concentrations against a wide spectrum of microorganisms, including but not limited to *S. epidermis, C. albicans, A. fumigatus,* etc. As previously indicated, the solutions contain as their principal microbiocide, a biguanide of formula (I). More particularly, within the formula I biguanides preferred are those wherein $R_1$ and $R_2$ substitutions when present are chlorine or bromine. Within this preferred group are compounds in which $R_1$ is hydrogen, $R_2$ is chlorine or bromine and X is a $C_1$—$C_3$ alkyl radical i.e. methyl, ethyl, propyl or isopropyl.

In addition to the foregoing, the present invention also includes water-soluble salts and free bases, as, for example, hydrochloride and borate salts, acetate, gluconate, sulfonate, tartrate and citrate salts.

Specific representative embodiments of biguanides for use in the present invention include free bases and water soluble salts wherein:

| $R_1$ | $R_2$ | X |
|---|---|---|
| H | Cl | $CH_3$— |
| H | Br | $CH_3$—$CH_2$— |
| Cl | H | H |
| H | H | H |
| H | H | $CH_3$— |

The monomeric biguanides may be prepared according to methods well documented in the literature. For example, Rose et al, *J. Chem. Soc.*, pp. 4422—4425 (1956) describe methods for the preparation of various bisdiguanides having antibacterial activity, including chlorhexidine. Generally, the compounds are prepared either by condensing one mole of a biscyanoguanidine with two moles of an amine hydrochloride, or conversely by the interaction of two moles of an N-arylcyanoguanidine with one of a diamine salt. The following reactions illustrate the general method for their preparation:

(A)

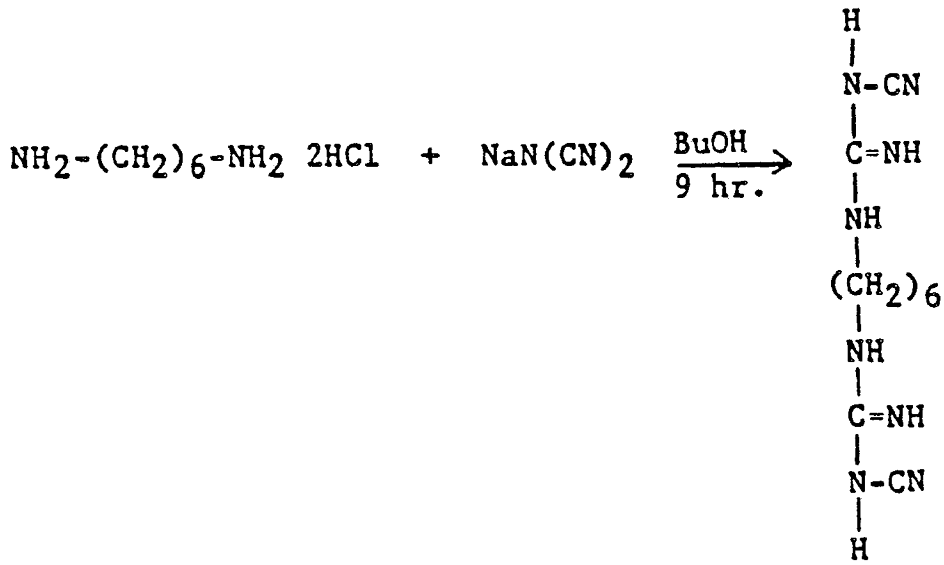


(I)

(B)

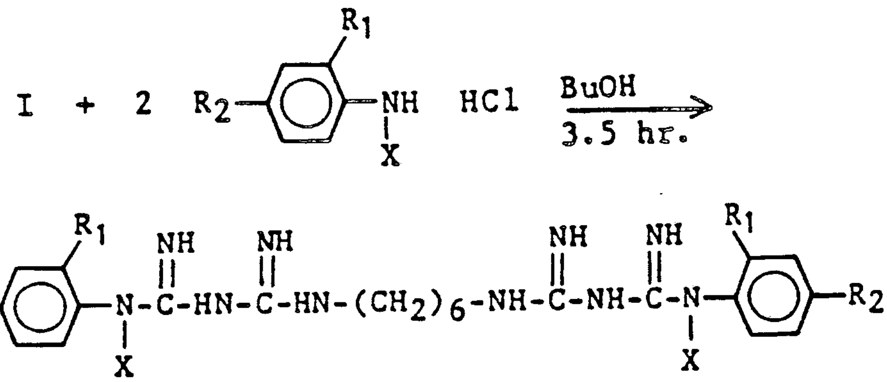


Reaction (A) involving the preparation of 1,6-di($N^3$-cyano-$N^1$-guanidino) hexane is carried out using a hexamethylene bridged system, 1,6-diaminohexane dihydrochloride, prepared by dissolving the appropriate amine in ether and then bubbling hydrochloric acid gas into the solution, precipitating out the amine salt which after being filtered is reacted with sodium dicyanimide dihydrochloride in n-butanol. The mixture is refluxed for up to 9 hours. The resultant product is isolated and purified. The product of reaction (A) is then reacted with the appropriate arylamine hydrochloride in reaction (B) to yield the bisbiguanide. Alternatively, the bisbiguanides may be condensed by heating the arylamine hydrochloride with the product of reaction (A) without solvent at 160°C for 2 hours.

The solutions are generally effective with biguanide concentrations as low as 0.00001 weight percent. More particularly, the disinfectant and preservative solutions may contain from 0.00001 to 7 weight percent of the active biguanide.

It has also been discovered that the microbicidal activity of the solutions may in some cases be enhanced or spectrum of activity broadened through the use of a potentiating amount of a second disinfectant or germicidal agent. Under such circumstances, the total concentration of disinfectant required when the biguanide is used in combination with other germicidal agents may be lowered further due to complementary microbicidal activity which is most desirable in achieving the lowest possible potential for lens binding, concentrating and eye tissue inflammation.

The second disinfectant/germicide can be employed as a solution preservative, but it may in some instances also function to potentiate, complement or broaden the spectrum of microbicidal activity of the biguanide disinfectant. This includes microbicidally effective amounts of germicides which are compatible with and do not precipitate in the presence of the biguanide, and comprises concentrations ranging from 0.00001 to 0.5 weight percent, and more preferably, from 0.0001 to 0.1 weight percent. Suitable complementary germicidal agents include, but are not limited to thiomersal, sorbic acid, 1,5-pentanedial, alkyl triethanolamines, phenylmercuric salts, e.g. nitrate, borate, acetate, chloride and mixtures thereof. Other germicidal compounds and salts may be used. Suitable salts are soluble in water at ambient temperature to the extent of at least 0.5 weight percent. These salts include the gluconate, isothionate (2-hydroxyethanesulfonate), formate, acetate, glutamate, succinamate, monodiglycollate, dimethanesulfonate, lactate, diisobutyrate and glucoheptonate.

Further embodiments of potentiating or complementary disinfecting agents for use with the biguanides also include certain quaternary ammonium compounds which possess a generally wide

spectrum of bactericidal activity and wetting properties. Representative examples of the quaternary ammonium compounds are compositions comprised of balanced mixtures of n-alkyl dimethyl benzyl ammonium chlorides and n-alkyl dimethyl ethylbenzyl ammonium chlorides. Such dual quaternary ammonium compositions are described in US—A—3,525,793 and 3,472,939 and are commercially available from Onyx Chemical Company, Jersey City, New Jersey, under the trademark BTC 2125M.

The aqueous solutions for use in the methods of the present invention are preferably adjusted with tonicity agents to approximate the osmotic pressure of normal lacrimal fluids which is equivalent to a 0.9 percent solution of sodium chloride or 2.5 percent of glycerol solution. Solutions are made substantially isotonic with physiological saline used alone or in combination, otherwise if simply blended with sterile water and made hypotonic the treated lenses may adhere tightly to the cornea. Correspondingly, excess saline may result in the formation of a hypertonic solution which will cause stinging and eye irritation.

The aqueous isotonic solutions with optional germicidal agents are useful disinfectants for both hard and soft contact lenses without any further additives. However, the solutions of the present invention may be formulated into specific contact lens care products, such as wetting solutions, soaking solutions, cleaning and conditioning solutions, as well as all purpose type lens care solutions by the addition of various buffering agents, optional cleaning and wetting agents, sequestering agents and viscosity builders and mixtures thereof. Such additives make the solutions in many instances more acceptable to the user in terms of greater comfort. However, the additives must be non-toxic and compatible with contact lenses.

Suitable buffers include, for example, sodium or potassium citrate, citric acid, boric acid, sodium bicarbonate and various mixed phosphate buffers, including combinations of $Na_2HPO_4$, $NaH_2PO_4$ and $KH_2PO_4$. Generally, buffers may be used in amounts ranging from about 0.05 to 2.5 percent, and more preferably, from about 0.1 to 1.5 percent (w/v).

When used, neutral or non-ionic surfactants impart cleaning and conditioning properties and are usually present in amounts up to 15 weight percent. The surfactant should be soluble in the lens care solution, non-irritating to eye tissues and will usually have a hydrophile-lipophile balance (HLB) of 12.4 to 18.8. Satisfactory non-ionic surfactants include polyethylene glycol esters of fatty acids, e.g. coconut, polysorbate, polyoxyethylene or polyoxypropylene ethers of higher alkanes ($C_{12}$—$C_{18}$). Examples of the preferred class includes polysorbate 20 (available under the trademark Tween 20), polyoxyethylene (23) lauryl ether (Brij® 35), polyoxyethylene (40) stearate (Myrj® 52), polyoxyethylene (25) propylene glycol stearate (Atlas® G 2612).

One non-ionic surfactant in particular consisting of a poly(oxypropylene)-poly(oxyethylene) adduct of ethylene diamine having a molecular weight from 7,500 to 27,000 wherein at least 40 weight percent of said adduct is poly(oxyethylene) has been found to be particularly advantageous for use in cleaning and conditioning both soft and hard contact lenses when used in amounts from 0.01 to 15 weight percent. Such surfactants are available from BASF-Wyandotte under the registered trademark — Tetronic.

In addition to the foregoing buffering agents, cleaning and wetting agents, in some instances it may be desirable to include sequestering agents in the contact lens care solution in order to bind metal ions which might otherwise react with protein deposits and collect on lens surfaces. Ethylenediaminetetraacetic acid (EDTA) and its salts (disodium) are preferred examples. They are usually added in amounts ranging from 0.1 to 2.0 weight percent.

It may also be desirable to include water-soluble viscosity builders in the biguanide containing lens disinfectant solutions. Because of their demulcent effect, viscosity builders have a tendency to enhance the lens wearer's comfort by means of a film on the lens surface cushioning impact against the eye. Included amoung the water-soluble viscosity builders are the cellulose polymers, like hydroxyethyl or hydroxypropyl cellulose, and carboxymethyl cellulose. Such viscosity builders may be employed in amounts ranging from 0.01 to 4.0 weight percent, or less.

The aqueous solutions can be effectively used in disinfecting contact lenses by any of the well recognized methods. For example, lenses may be treated by the "cold" soaking method at room temperature for a time period sufficient to disinfect the lenses which is usually a period ranging from 4 to 12 hours. The lenses are then removed from the solution, washed in preserved isotonic saline solution and then replaced on the eyes.

In addition to the cold soaking method, the solutions disclosed herein are adaptable for use in most types of disinfecting equipment, such as ultrasonic cleaners. Because the solutions are also stable when heated, they are adaptable for use with high temperature disinfecting methods. Typically, lenses are heated in the range of 80° to 90°C in a disinfecting unit containing the solution for a time period of at least 10 minutes, removed and rinsed with isotonic saline.

The following specific examples illustrate the invention.

## Example I

Synthesis of 1,6-di($N^3$-Cyano-$N^1$-guanidino)hexane

94.5 g (0.5 mole) of 1,6-diaminohexane dihydrochloride mixed with 103 g (1 mole) of sodium dicyanimide is added to 700 ml of n-butanol. The mixture is refluxed with stirring for 8.5 hours. The reaction mixture is then cooled with ice. The product is precipitated, filtered off, washed with ice water and dried. The product has a melting point of 200—203°C. It is recrystallized from aqueous methanol (1.5 liter of methanol plus 2.5 liter of water). The above-identified material thus obtained (116.5 g, 0.47 mole, 94%

yield) had a m.p. 206—208°C.

Example II

Synthesis of N,N[1]-bis(o-chlorophenyl)-3,12-diimino-2,4,11,13 tetraaza-tetradecane diimidamide dihydrochloride

6.0 g (0.024 mole) of the product of Example I and 8.0 g (0.048 mole) of ortho-chloroaniline hydrochloride are added to 60 ml of 2-ethoxyethanol and the whole mixture is refluxed with stirring for 11.5 hours. On cooling, a small amount of material is separated with a m.p. >300°C, which is rejected. The filtrate is evaporated to dryness (rotavap) and the residue consisting of 1.26 g (0.0022 mole) is recrystallized from water (25 ml) and yields 1.0 g (7% yield) of the above-identified product having a m.p. 231—234°C.

Example III

Synthesis of N,N[1]-dimethyl-bis(p-chlorophenyl)-3,12-diamino-2,4,11,13 tetraaza-tetradecane diimidamide dihydrochloride

6.0 g (0.024 mole) of the product of Example I and 8.6 g (0.048 mole) of N-methyl-p-chloroaniline hydrochloride are heated in an oil bath at 160°C (± 5°C) for 2 hours. The mixture is cooled and dissolved in 50 ml of ethanol to which is added 50 ml of ethyl acetate. The crude precipitate weighs 5.5 g (0.009 mole) and melts at 159—162°C. After recrystallization from water, it is obtained as 3.3 g (0.005 mole, 21% yield) having a m.p. 171—173°C.

Example IV

An aqueous contact lens disinfecting solution is prepared having the following formulation:

| | percent (w/v) |
|---|---|
| N,N[1]-Bis(o-chlorophenyl)-3,12-diimino-2,4,11,13 tetraaza-tetradecane diimidamide dihydrochloride | 0.0025 |
| Boric acid and sodium borate to maintain pH 7.2 $Na_2$ EDTA | 0.103 |
| Tonicity adjusted with isotonic saline containing 0.9% NaCl *Dist. water qs* | 100.0 |

The solution is prepared by dissolving the sodium borate in approximately 80 percent of the distilled water. The disodium EDTA is then added to the sodium borate solution, followed by dissolving the boric acid and sodium chloride therein. The biguanide is then added followed by the balance of the distilled water. The solution is sterilized by forcing through a 0.22 μm (micron) cellulose acetate filter by means of a peristaltic pump and packaged in sterilized plastic containers.

Example V

A group of aqueous biguanide solutions are prepared most with concentrations of 0.1; 0.01 and 0.001 percent (w/v). *In vitro* microbicidal activity of the solutions is determined by exposing *S. epidermidis, C. Albicans* and *A. fumigatus* to 20 ml of each solution at room temperature for 5 hours. Subsequently, an aliquot sample of each is placed on an agar plate and incubated for 48 hours at elevated temperatures. At the conclusion of the incubation period the plates are examined for the development of colonies. The following table provides the results of the *in vitro* studies.

TABLE

| DISINFECTANT/ PRESERVATIVE | CONCENTRATION-% (w/v) | *S. epidermidis* (Average Log Reduction) | *C. albicans* (Average Log Reduction) | *A. fumigatus* (Average Log Reduction) |
|---|---|---|---|---|
| N,N'-Bis(o-chlorophenyl)-3,12-diimino-2,4,11,13-tetraaza-tetradecane diimidamide·2 HCl | 0.1 | 6.5 | 4.8 | 1.6 |
| | 0.01 | 6.0 | 4.5 | 1.8 |
| | 0.001 | 6.1 | 3.2 | 0.7 |
| N,N'-dimethyl-bis(p-chlorophenyl)-3,12-diimino-2,4,11,13-tetraaza-tetradecane diimidamide·2 HCl | 0.1 | 6.5 | 6.0 | 1.4 |
| | 0.01 | 6.0 | 6.1 | 1.9 |
| | 0.001 | 4.2 | 1.5 | 0.9 |
| N,N'-bisphenyl-3,12-diimino-2,4,11,13-tetraaza-tetradecane diimidamide·2 HCl | 0.1 | 6.4 | 6.0 | 2.0 |
| | 0.01 | 6.0 | 4.8 | 2.4 |
| | 0.001 | 3.3 | 1.6 | 0.6 |
| N,N'-bis(p-chlorophenyl)-3,12 diimino-2,4,11,13-tetraaza-tetradecane diimidamide·2 HCl (chlorhexidine dihydrochloride) | 0.001 | 3.7 | 0.3 | 0.1 |

The foregoing *in vitro* studies demonstrate the improved antimicrobial activity of biguanide compounds of formula I as compared with chlorhexidine.

**Claims**

1. A method of disinfecting and/or preserving contact lenses which comprises treating said lenses with an antimicrobial amount of a solution comprising a biguanide or its water-soluble salt, characterized in that said biguanide has the formula:

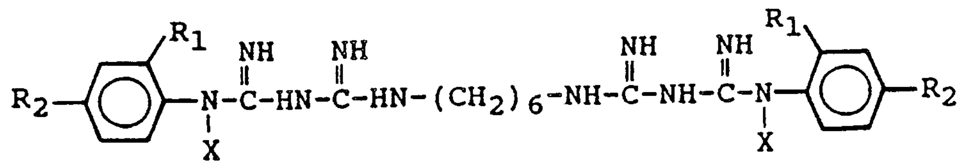

(I)

wherein $R_1$ and $R_2$ are hydrogen or halogen, and X is hydrogen or $C_1$—$C_3$ alkyl, provided that when $R_2$ is halogen then $R_1$ is hydrogen and X is $C_1$—$C_3$ alkyl.

2. A method according to Claim 1, wherein $R_1$ and $R_2$ are hydrogen, chlorine or bromine.

3. A method according to Claim 1, wherein $R_1$ is halogen and $R_2$ is hydrogen.

4. A method according to Claim 3, wherein $R_1$ is chlorine, and $R_2$ and X are each hydrogen.

5. A method according to Claim 1, wherein $R_1$, $R_2$ and X are hydrogen.

6. A method according to Claim 1, wherein $R_1$ is hydrogen, $R_2$ is halogen and X is $C_1$—$C_3$ alkyl.

7. A method according to Claim 6, wherein $R_2$ is chlorine and X is $C_1$—$C_3$ alkyl.

8. A method according to any preceding claim, wherein the biguanide-containing solution includes a tonicity and a buffering agent.

9. A method according to any preceding claim, wherein the solution comprises a microbially effective mixture of said biguanide and at least one other germicidal agent.

10. A method according to Claim 9, wherein said other germicidal agent is selected from thiomersal, sorbic acid and phenylmercuric salts.

11. A contact lens cleaning or disinfecting solution comprising a biguanide or its water soluble salt, characterized in that said biguanide is as defined in any one of Claims 1—7.

12. A solution according to Claim 11, containing from 0.00001 to 7 weight percent of said biguanide.

13. A solution according to Claim 11 or Claim 12 and comprising a microbially effective mixture of said biguanide and at least one other germicidal agent.

14. A solution according to Claim 13, wherein said other germicidal agent is selected from thiomersal, sorbic acid and phenylmercuric salts.

15. A solution according to any one of Claims 11—14, and including also tonicity and buffering agents.

**Patentansprüche**

1. Verfahren zur Desinfektion und/oder Konservierung von Kontaktlinsen umfassend die Behandlung der genannten Linsen mit einer antimikrobiellen Menge einer Lösung, die ein Biguanid oder sein wasserlösliches Salz enthält, dadurch gekennzeichnet, daß das genannte Biguanid die Formel

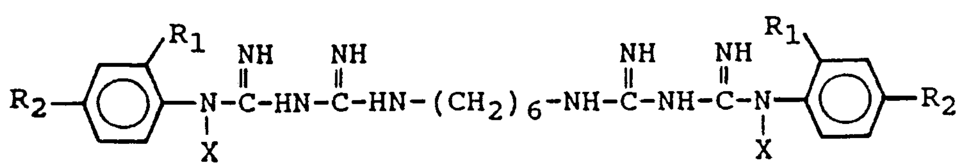

(I)

aufweist, worin $R_1$ und $R_2$ Wasserstoff oder Halogen bedeuten und X für Wasserstoff oder $C_1$ bis $C_3$-Alkyl steht, mit der Maßgabe, daß, wenn $R_2$ Halogen ist, $R_1$ Wasserstoff darstellt und X $C_1$ bis $C_3$-Alkyl ist.

2. Verfahren nach Anspruch 1, worin $R_1$ und $R_2$ Wasserstoff, Chlor oder Brom darstellen.

3. Verfahren nach Anspruch 1, worin $R_1$ Halogen ist und $R_2$ Wasserstoff bedeutet.

4. Verfahren nach Anspruch 3, worin $R_1$ für Chlor steht und $R_2$ und X jeweils Wasserstoff darstellen.

5. Verfahren nach Anspruch 1, worin $R_1$, $R_2$ und X Wasserstoff sind.

6. Verfahren nach Anspruch 1, worin $R_1$ Wasserstoff bedeutet, $R_2$ Halogen ist und X $C_1$—$C_3$-Alkyl darstellt.

7. Verfahren nach Anspruch 6, worin $R_2$ Chlor darstellt und X für $C_1$—$C_3$-Alkyl steht.

8. Verfahren nach einem der vorhergehenden Ansprüche, worin die Biguanid enthaltende Lösung ein Mittel zur Einstellung der Tonizität und einen Puffer enthält.

9. Verfahren nach einem der vorhergehenden Ansprüche, worin die Lösung eine mikrobiell wirksame Mischung des genannten Biguanids und wenigstens eines anderen germiziden Wirkstoffs enthält.

10. Verfahren nach Anspruch 9, worin der genannte andere germizide Wirkstoff aus Thiomersal,

zorbinsäure und Phenylquecksilber-II-salzen gewählt ist.

11. Kontaktlinsen-Reinigungs- oder Desinfektionslösung enthaltend ein Biguanid oder sein wasserlösliches Salz, dadurch gekennzeichnet, daß das genannte Biguanid die in einem der Ansprüche 1 bis 7 definierte Bedeutung besitzt.

12. Lösung nach Anspruch 11, mit einem Gehalt von 0,00001 bis 7 Gew.-% des genannten Biguanids.

13. Lösung nach Anspruch 11 oder 12 und enthaltend eine mikrobiell wirksame Mischung aus dem genanntn Biguanid und wenigstens einem anderen germiziden Wirkstoff.

14. Lösung nach Anspruch 13, worin der genannte andere germizide Wirkstoff aus Thiomersal, Sorbinsäure und Phenylquecksilber-II-salzen gewählt ist.

15. Lösung nach einem der Ansprüche 11 bis 14, weiters enthaltend Mittel zur Einstellung der Tonizität und Puffer.

**Revendications**

1. Un procédé de désinfection et/ou de conservation de lentilles de contact, qui comprend le traitement desdites lentilles avec une quantité antiseptique d'une solution comprenant une biguanidine ou son sel soluble dans l'eau, caractérisé en ce que ladite biguanidine est de la formule suivante:

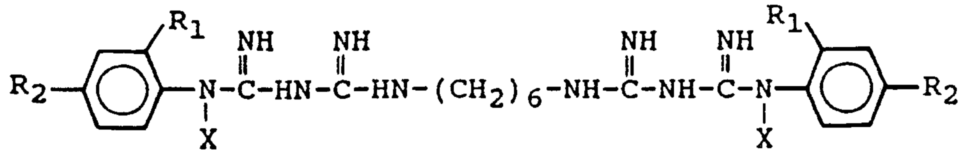

(I)

dans laquelle $R_1$ et $R_2$ sont de l'hydrogène ou de l'halogène, et X est de l'hydrogène ou un alkyle en $C_1$—$C_3$, compte tenu que, lorsque $R_2$ est un halogène, $R_1$ est de l'hydrogène et X un alkyle en $C_1$—$C_3$.

2. Un procédé selon la revendication 1, dans lequel $R_1$ et $R_2$ sont de l'hydrogène, du chlore, ou du brome.

3. Un procédé selon la revendication 1, dans lequel $R_1$ est un halogène et $R_2$ est de l'hydrogène.

4. Un procédé selon la revendication 3, dans lequel $R_1$ est du chlore, et $R_2$ et X sont chacun de l'hydrogène.

5. Un procédé selon la revendication 1, dans lequel $R_1$ et X sont de l'hydrogène.

6. Un procédé selon la revendication 1, dans lequel $R_1$ est de l'hydrogène, $R_2$ est un halogène et X est un alkyle en $C_1$—$C_3$.

7. Un procédé selon la revendication 6, dans lequel $R_2$ est du chlore et X est un alkyle en $C_1$—$C_3$.

8. Un procédé selon une quelconque des revendications précédentes, dans lequel la solution contenant la biguanidine comprend un agent actif et un agent tampon.

9. Un procédé selon une quelconque des revendications précédentes, dans lequel la solution comprend un mélange efficace du pont de vue antiseptique de ladite biguanidine et au moins un autre agent germicide.

10. Un procédé selon la revendication 9, dans lequel ledit autre agent germicide est choisi parmi l'acide thiomersalique, sorbique, et les sels phenylmercuriques.

11. Une solution pour nettoyer ou désinfecter des lentilles de contact comprenant une biguanidine ou son sel soluble dans l'eau, caractérisé en ce que ladite biguanidine est telle que définie dans l'une quelconque des revendications 1 à 7.

12. Une solution selon la revendication 11, contenant de 0,00001 à 7 pour cent en poids de ladite biguanidine.

13. Une solution selon la revendication 11, ou revendication 12, et comprenant un mélange efficace du point de vue antiseptique de ladite biguanidine et au moins un autre agent germicide.

14. Une solution selon la revendication 13, dans laquelle ledit autre agent germicide est choisi parmi l'acide thiomersalique, l'acide sorbique, et les sels phenylmercuriques.

15. Une solution selon une quelconque des revendications 11 à 14, et comprenant également des agents actifs et tampon.